# EUROPEAN PATENT APPLICATION

(11) **EP 2 422 767 A2**
(43) Date of publication of application: **29.02.2012**
(21) Application number: 11190638.4
(22) Date of filing: 29.04.2005
(51) Int. Cl.: A61K 9/00, A61K 31/40

(54) **Methods for preparing dry powder compositions of glycopyrrolate**

(30) Priority: 30.04.2004 GB 0409703
(62) Divisional of application: 05738040.4
(71) Applicant: Vectura Limited, Chippenham, Wiltshire SN14 6FH (GB)
(72) Inventor: Morton, David, Chippenham, Wiltshire SN14 6FH (GB); Shott, Martin, Chippenham, Wiltshire SN14 6FH (GB); Davies, Rebecca, Chippenham, Wiltshire SN14 6FH (GB)
(74) Representative: Stephen, Robert John

(57) **Abstract**

The present invention relates to pharmaceutical compositions comprising the antimuscarinic agent glycopyrrolate, for example the salt glycopyrronium bromide. In particular, the present invention relates to dry powder compositions which exhibit improved stability over time, and methods for producing the same.

## Description

The present invention relates to pharmaceutical compositions comprising the antimuscarinic agent glycopyrrolate, for example the salt glycopyrronium bromide. In particular, the present invention relates to dry powder compositions which exhibit improved stability over time, and methods for producing the same.

Glycopyrrolate is an antimuscarinic agent which is useful in the treatment of conditions such as chronic obstructive pulmonary disease (COPD), asthma, cystic fibrosis (CF) and related airway diseases. It is known to provide glycopyrrolate formulations in the form of dry powder formulations, for administration using dry powder inhalers. Frequently salts of glycopyrrolate are used, such as glycopyrronium bromide.

The term "glycopyrrolate" as used in connection with the present invention is intended to encompass salt forms or counterion formulations of glycopyrrolate, such as glycopyrrolate bromide, as well as isolated stereoisomers and mixtures of stereoisomers. Derivatives of glycopyrrolate are also encompassed.

WO 01/76575 discloses the delivery of glycopyrrolate by dry powder inhaler. The formulation disclosed in this application may include magnesium stearate to improve dispersion of the dry powder and to help prolong the therapeutic effect by providing a controlled release of the glycopyrrolate. Studies show that this formulation may exert its therapeutic effect for more than or less than 12 hours. WO 01/76575 also discloses the use of magnesium stearate applied in a specific manner to the surface of micronised glycopyrrolate particles, for subsequent use in an inhaled formulation with delayed release properties.

WO 00/28979 briefly discloses an example of a dry powder composition including a combination of 0.2% w/w formoterol and 0.5% w/w glycopyrrolate and including 0.5% w/w magnesium stearate conventionally blended in a tumble mixer with a lactose carrier (98.8% w/w). It is alleged that the magnesium stearate protects the formulation from the deleterious effects of moisture ingress.

WO 96/23485, WO 01/78694, WO 01/78695, WO 02/43701 and WO 02/00197 all disclose the use of magnesium stearate with any dry powder inhaled system for improving the dispersibility of the micronised drug particles from the formulation, in comparison to a formulation in the absence of such an additive. Additive materials which improve the dispersibility of the drug particles are often referred to as force control agents.

However, during development work with dry powder formulations for use in dry powder inhalers for the treatment of COPD, asthma, CF and related airway diseases, it has been found that the above disclosures do not teach the satisfactory production of a robust and stable dry powder formulation of glycopyrrolate.

It has been found that glycopyrrolate which is generated as a micronised powder as taught in the prior art suffers from stability problems on storage, even where the formulation includes an additive material for improving dispersibility or for protecting against moisture, such as magnesium stearate, as disclosed in WO 00/28979.

Indeed, glycopyrrolate has been found to have an acute problem with respect to its stability, especially immediately following a conventional micronisation process. Micronisation of any drug, and specifically here glycopyrrolate, may involve the injection of a relatively coarse source powder into a system which involves multiple high-speed collisions. Typically source powders of un-micronised drug will exist in particle sizes substantially greater than 10µm. The objective of the micronisation process is to reduce the primary particle size to a size which is small enough to be delivered to the respiratory airways. For example, it is known that a suitable size may be 10 to 0.1µm, and preferably 6 to 0.1µm or 5 to 0.5µm.

The multiple high-speed collisions are employed in micronisation to provide the milling action required to break the particles down to the required size. It is also well known that such milling action may also induce the generation of non-crystalline material, especially on the surface of the particles. Such non-crystalline material may be amorphous material.

It has been found from studies of glycopyrronium bromide powder that the presence of non-crystalline or amorphous glycopyrronium bromide material can lead to significant physical instability. This instability appears due to the aggressive uptake of water by the amorphous fraction, leading to partial dissolution, and subsequent re-crystallization. Amorphous glycopyrrolate appears to aggressively take up water when stored at relative humidities as low as 30%, indicating that the amorphous glycopyrrolate is inherently unstable even in conditions which are normally considered to be "dry" conditions. Indeed, the uptake of only a very small amount of water (as little as approximately 4%) is believed to be sufficient to cause re-crystallisation. Thus, glycopyrrolate is extremely unstable compared to the majority of active agents, including those that are generally considered to be sensitive to moisture.

100% amorphous glycopyrrolate was obtained by lyophilisation. This amorphous glycopyrrolate was found to be very hygroscopic. Storing this amorphous glycopyrrolate at ambient atmosphere (30-50% RH (relative humidity)/ 21-25°C) resulted in its transformation into a very sticky mass within minutes. Confirmation of this hygroscopicity (at RH >0%) was obtained by DVS (dynamic vapour sorption), which is a moisture sorption analysis, and after the experiment the amorphous was found to be crystalline and was a sintered solid.

The glass transition temperature by DSC (differential scanning calorimetry) of a dry amorphous glycopyrrolate sample was at 65°C. It is known from many substances that water acts as a plasticizer, i.e., it depresses the glass transition temperature. It is anticipated that in this case the glass transition may be depressed to below room temperature (at as little as 30-40% RH) and that crysytallization occurs. Prior to crystallization the sample becomes sticky. Consequently, it was concluded that recrystallized parts which were previously amorphous will act as a form of glue between crystalline parts analogous to a sintering process.

Similarly, amorphous glycopyrrolate was formed by spray drying a 1% solution of the drug in water using a Büchi laboratory spray dryer. Immediately on collection of the powder within the collection cyclone, the powder formed a wet slurry and no dry powder could be recovered.

In a relatively short period of time, compared to that demanded for storage of an inhaled product, moisture can be drawn in by the non-crystalline material in a dry powder glycopyrrolate formulation, even in conditions which are generally considered to be relatively dry. The moisture absorption leads to the production of an intermediate wet form, followed by re-crystallization and possibly the release of any surplus moisture not required by the newly formed crystalline structure. This process is likely to induce the formation of solid bridges at contact points between the particles present. Where these bridges form, it has been found that they may be strong enough to result in a significant reduction in the powder dispersibility.

It is therefore an aim of the present invention to provide a dry powder composition comprising glycopyrrolate which exhibits greater stability than conventional dry powder glycopyrrolate formulations. It is also an aim of the present invention to provide methods for consistently and reliably preparing stable dry powder compositions comprising glycopyrrolate.

According to one aspect of the present invention, a dry powder formulation comprising glycopyrrolate is provided which is stable for a period of at least 1 year, more preferably a period of at least 2 years and most preferably a period of at least 3 years.

The glycopyrrolate may be a salt, isomer or derivative of glycopyrrolate, or mixtures thereof. In one embodiment, the glycopyrrolate is not R,R-glycopyrrolate.

The stability of a composition should be indicated by consistent dispersability of the powder over these periods, which may, for example, be measured in terms of a consistently good fine particle fraction or fine particle dose over time. In one embodiment of the invention, the fine particle fraction (<5µm) is consistently greater than about 30% over a period of at least 1 year, at least 2 years or at least 3 years when stored at normal temperatures and humidities for pharmaceutical products. In another embodiment of the invention, the fine particle fraction (<5µm) is consistently greater than about 40% over a period of at least 1 year, at least 2 years or at least 3 years. Preferably, the fine particle fraction (<5µm) is consistently greater than 30% or greater than 40% when the formulations are stored under standard testing conditions, such as 25°C/60% RH, 30°C/60% RH, 40°C/70% RH or 40°C/75% RH.

Preferably, the fine particle fraction of the dry powder formulations of the present invention is consistently at least about 30%, at least about 40%, at least about 50%, at least about 60%, at least about 70% or at least about 80%.

Preferably, the fine particle dose of the dry powder formulations of the present invention is consistently at least about 30%, at least about 40%, at least about 50%, at least about 60%, at least about 70% or at least about 80%.

In another embodiment of the invention, the dry powder formulations are packaged for storage and/or delivery by a dry powder inhaler and the packaged formulations are stable for at least 1, 2 or 3 years when stored at normal temperatures and humidities, i.e. the packaged formulations or products comprising the formulations do not have to be stored in a controlled environment in order to exhibit the desired stability.

As the instability of the conventional glycopyrrolate formulations appears to be due to moisture absorption, there are a number of measures which are proposed to increase stability.

Firstly, the amorphous content of the glycopyrrolate is to be reduced by improving the processing of the glycopyrrolate. Where the glycopyrrolate is micronised, the micronisation process may be improved, for example, by adjusting the conditions under which the milling takes place, to prevent the formation of amorphous material. Additionally or alternatively, the micronised product may be "conditioned" to remove the amorphous material.

Alternatively, the particles of glycopyrrolate may be engineered so that they include little or no amorphous material. Suitable methods for doing this are known to those skilled in the art. For example, glycopyrrolate powders with low non-crystalline content may be made using methods such as supercritical fluid processing using carbon dioxide, or other controlled forms of crystallisation or precipitation, such as slow precipitation, by emulsion methods, sono-crystallisation and the like.

Secondly, the exposure of the dry powder formulation to moisture when the powder is stored is preferably reduced. In this regard, it is particularly desirable to reduce exposure of the formulation to moisture during storage in capsules or blisters.

Finally, the inclusion of additive materials in the dry powder formulation can enhance the powder dispersability and protect the formulation from the ingress of moisture.

Batches of micronised glycopyrrolate were obtained and, following sealed storage for several weeks, the physical changes of the material from fine cohesive powders to solid agglomerates were observed.

The following section summarises the tests conducted on reported batches of glycopyrrolate received following micronisation:
Batch A:
   Micronised at 0.5kg/hr
   Injection pressure: 10 bar
   Micronisation pressure: 7 bar
   Sympatec sizing: d10 0.7µm, d50 1.8µm, d90 3.6µm
   Loss on drying: 0.7%
   DVS indicated crystalline material. On storage, soft lumps of material were found in bulk powder, and repeated particle sizing gave d50 values ranging between 2.6 and 3.5µm.
Batch B:
   Micronised at 0.5kg/hr
   Injection pressure: 10 bar
   Micronisation pressure: 7 bar
   Sympatec sizing: d10 1.0µm, d50 2.4µm, d90 4.8µm
   Loss on drying: 0.6%
   Water activity: 54% RH
   DVS indicated amorphous material was present. On storage, large hard lumps of material were found, and repeated particle sizing gave d50 values ranging between 36 and 160µm.
Batch C:
   Micronised at 0.4kg/hr
   Injection pressure: 10 bar
   Micronisation pressure: 9.8 bar
   Sympatec sizing: d10 0.8µm, d50 2.3µm, d90 4.8µm
   Loss on drying: 0.4%
   DVS indicated amorphous material was present. On storage, large hard lumps of material were found in bulk powder, and repeated particle sizing gave d50 value of 51µm.
Remicronised Batch C:
   Micronised at 0.5kg/hr
   Injection pressure: 10 bar
   Micronisation pressure: 9 bar
   Sympatec sizing: d10 1.0µm, d50 2.4µm, d90 4.5µm
   Loss on drying: 0.5%
   On storage, only soft lumps of material were found in bulk powder.

This summary shows that selected batches of micronised glycopyrrolate had formed hard agglomerates, and this appears to be associated with the presence of amorphous material, as the first batch, which contained no detectable amorphous material, exhibited good powder properties following storage. Consequently, it is believed that the formation of hard agglomerates occurs within a micronised powder that contains surface non-crystalline material, whether formulated with excipient, any moisture protection agent, a force control agent, or on its own.

The amorphous material will be located on the surface to have the greatest effect of this kind. The quantity of amorphous material relative to the bulk mass may be very small, as long as it is sufficient to produce this effect. The non-crystalline material will draw moisture from its surroundings. Sources of moisture may include the surrounding air or gas, the surrounding excipients or additives (such as lactose or force control agents), the packaging or device, such as a gelatin or other capsule material, or a plastic.

Tests have shown that all micronised glycopyrronium bromide prototype formulations made using conventional methods, including those that comprise additives (including magnesium stearate), disclosed in the prior art as noted above, have been found to degrade or deteriorate in aerosolisation performance over a period of 6 months. This deterioration has even been found to occur when stored under dry conditions. Deterioration in performance has been seen to be approximately 30 to 50% of original performance or more. Such deterioration would make these formulations unattractive for commercial use.

It has been suggested that conducting micronisation under the use of humidified air or other gas may help to reduce the generation of amorphous materials. Both WO 99/54048 and WO 00/32165 disclose that milling under increased humidity can reduce the generation of amorphous material. WO 00/32313 discloses the milling of material at reduced temperature using helium or a mixture of helium and another gas in order to reduce the formation of amorphous material. It should be noted that none of these prior art documents disclose that the milling of glycopyrrolate under these special conditions is beneficial.

However, the milling conditions disclosed in the prior art are not standard in micronisation practice and it may well prove to be difficult to control these processes. It may also prove difficult to use such processes on a commercial scale. Finally, the extent to which such processes may help to control the generation of amorphous material for the specific problem of glycopyrrolate is also not known. As mentioned above, glycopyrrolate presents particular problems because of its inherent instability.

In accordance with one embodiment of the present invention, the dry powder formulation comprising glycopyrrolate is prepared using a process, preferably a micronisation process, which is carried out under conditions which reduce the formation of amorphous material. Examples of suitable micronisation conditions include increased relative humidity (for example 30-70%) or micronisation using helium at reduced temperatures.

In another embodiment, the dry powder formulation comprising glycopyrrolate is micronised and then undergoes a "conditioning" step to remove or reduce the amorphous material content. Such conditioning steps include exposure to moisture to encourage re-crystallisation of the amorphous material without the formation of hard agglomerates. Examples of such conditioning are discussed in more detail below.

It is known for gelatin capsules to contain in the order of 10 to 15% water, and for this to provide a sufficient source of water to create a moisture instability problem. The moisture content of the gelatin capsules has been shown to drop as the water is extracted by the capsule contents. The water content in the gelatin capsules acts as a plasticizer so that when the water is extracted and the water content drops, the capsules become more brittle, which will affect piercing and the like.

A recent article on improvements in hypromellose capsules (B. E. Jones, Drug Delivery Technology, Vol 3 No. 6, page 2, 2003), describes the problems associated with gelatin capsules for use in dry powder inhalers. These problems include changes in brittleness and hence piercing consistency, and related dispersion performance as a function of the changes in gelatin moisture content. The potential of the gelatin to act as a moisture source, which can be released to the powdered contents of the capsule, is also discussed, as are the variations in electrostatic charge properties.

Capsules can be made with hypromellose (HPMC) or other celluloses or cellulose derivatives which do not rely on moisture as a plasticizer. The moisture content of such capsules can be less than 10%, or even below 5% or 3%, and this makes such capsules more suitable for use with glycopyrrolate.

Capsules can also be made from gelatin containing one or more plasticizers other than water, such as PEG, glycerol, sorbitol, propyleneglycol or other similar polymers and co-polymers, hence allowing the moisture content to be reduced to below 10 %, or even below 5% or 3%.

Alternatively, capsules can be made from synthetic plastics or thermoplastics (polyethylene or polycarbonate or related plastics) containing reduced moisture content below 10 %, or even below 5% or 3%. Further alternative capsules with reduced moisture content are made from starch or starch derivatives or chitosan.

In the foregoing capsules, the problem of brittleness is reduced. Furthermore, capsules such as those made from celluloses have been found to pierce more consistently and reliably, and the pierce hole made appears to be more cleanly formed and spherical, with less shedding of particles. The aerosolisation of the powder contents has also been found to be improved, as well as being more consistent.

In an further approach to solving the problem of moisture absorption by dry powder glycopyrrolate formulations, an inhaler device is used which includes a means for protecting the formulation from moisture, for example within a sealed blister, such as a foil blister, with suitable sealing to prevent the ingress of moisture. Such devices are known, for example the GyroHaler (Vectura) or Diskus (GSK) devices. It is believed to be particularly advantageous if the blister is pierced using a simple mechanism, such as with the GyroHaler. This device has been developed by Vectura and it is an inhalation device for oral or nasal delivery of a medicament in powdered form. The powdered medicament is stored in a strip of blisters and each blister has a puncturable lid. When the inhaler is to be used, the lid of the aligned blister is punctured, thereby allowing an airflow through the blister to be generated to entrain the dose contained therein and to carry the dose out of the blister and into the user's airway via the inhaler mouthpiece. This arrangement with blisters having puncturable lids allows the blisters to have the best possible seal. In contrast, in blister systems such as the Diskus where the lids of the blisters are peeled open, it is more difficult to maintain an optimum seal due to the restrictions on the nature of the bond required to allow peeling to occur.

Thus, in a further embodiment of the present invention, the dry powder formulation comprising glycopyrrolate is stored in packaging made from a material which itself has a moisture content of less than 10%, preferably less than 5% and more preferably less than 3%.

The packaging should also preferably prevent the ingress of moisture, so that the powder is protected from external sources of moisture. Foil sealed blisters are en example of a packaging which prevents ingress of moisture.

In this latter regard, the prevention of the ingress of moisture from external sources may be assisted by further packaging. For example, HPMC capsules may be stored in a sealed environment, such as an additional layer of foil packaging.

In an alternative embodiment, the dry powder formulation is dispensed from a multidose dry powder inhaler device wherein the powder is stored in a reservoir as opposed to individually packaged doses. In such an embodiment, the device should offer superior moisture protection compared to conventional reservoir devices. For example, the device should include one or more of the following features: a sealed reservoir chamber (for example including a sealing gasket to seal the reservoir chamber), plastics materials exhibiting very low moisture permeability (for forming the walls of the reservoir chamber), and a desiccant.

In a yet further embodiment of the present invention, the dry powder formulation comprising glycopyrrolate further comprises an additive material, such as a so-called force control agent. A force control agent is an agent which reduces the cohesion between the fine particles within the powder formulation, thereby promoting deagglomeration upon dispensing of the powder from the dry powder inhaler. Suitable force control agents are disclosed in WO 96/23485 and they preferably consist of physiologically acceptable material, despite the fact that the material may not always reach the lung.

The force control agent may comprise or consist of one or more compounds selected from amino acids and derivatives thereof, and peptides and derivatives thereof, the peptides preferably having a molecular weight from 0.25 to 1000Kda. Amino acids, peptides and derivatives of peptides are physiologically acceptable and give acceptable release or deagglomeration of the particles of active material on inhalation. Where the force control agent comprises an amino acid, it may be one or more of any of the following amino acids: leucine, isoleucine, lysine, valine, methionine, and phenylalanine. The force control agent may be a salt or a derivative of an amino acid, for example aspartame or acesulfame K. The D-and DL-forms of amino acids may also be used.

The force control agents may include one or more water soluble substances. This helps absorption of the force control agent by the body if it reaches the lower lung. The force control agent may include dipolar ions, which may be zwitterions. It is also advantageous to include a spreading agent as a force control agent, to assist with the dispersal of the composition in the lungs. Suitable spreading agents include surfactants such as known lung surfactants (e.g. ALEC, Registered Trade Mark) which comprise phospholipids, for example, mixtures of DPPC (dipalmitoyl phosphatidylcholine) and PG (phosphatidylglycerol). Other suitable surfactants include, for example, dipalmitoyl phosphatidylethanolamine (DPPE), dipalmitoyl phosphatidylinositol (DPPI).

The force control agent may comprise a metal stearate, or a derivative thereof, for example, sodium stearyl fumarate or sodium stearyl lactylate. Advantageously, it comprises a metal stearate. For example, zinc stearate, magnesium stearate, calcium stearate, sodium stearate or lithium stearate. Preferably, the additive material comprises or consists of magnesium stearate.

The force control agent may include or consist of one or more surface active materials, in particular materials that are surface active in the solid state, which may be water soluble or water dispersible, for example lecithin, in particular soya lecithin, or substantially water insoluble, for example solid state fatty acids such as oleic acid, lauric acid, palmitic acid, stearic acid, erucic acid, behenic acid, or derivatives (such as esters and salts) thereof such as glyceryl behenate. Specific examples of such materials are phosphatidylcholines, phosphatidylethanolamines, phosphatidylglycerols and other examples of natural and synthetic lung surfactants; lauric acid and its salts, for example, sodium lauryl sulphate, magnesium lauryl sulphate; triglycerides such as Dynsan 118 and Cutina HR; and sugar esters in general. Alternatively, the force control agent may be cholesterol.

Other possible force control agents include sodium benzoate, hydrogenated oils which are solid at room temperature, talc, titanium dioxide, aluminium dioxide, silicon dioxide and starch. Also useful as force control agents are film-forming agents, fatty acids and their derivatives, as well as lipids and lipid-like materials.

Force control agents which are particularly suitable for use in the present invention include magnesium stearate, amino acids including leucine, lysine, arginine, histidine, cysteine and their derivatives, lecithin and phospholipids. The inclusion of these force control agents is expected to improve the efficacy of the glycopyrrolate for treating respiratory disorders such as COPD, asthma or CF.

Further, it is believed to be important for any force control agent to be predominantly present on the surface of the glycopyrrolate particles, as well as or rather than being on the surface of the carrier particles. It has been found that a high shear blending method is advantageous to achieve this.

In addition to reducing the cohesion between the fine particles of the glycopyrrolate formulation, additive materials, including the force control agents mentioned above, may have further benefits when used in the present invention. It has been suggested that some force control agents, such as magnesium stearate, are able to themselves reduce the ingress of moisture into the dry powder formulation. Furthermore, many force control agents act as surfactants. When these agents are administered to the lung, they tend to rapidly spread over the surface of the lung. It is postulated that this rapid dispersion of the surfactants may well assist in the dispersion of the glycopyrrolate in the formulation, thereby assisting and enhancing its therapeutic effect.

From the foregoing it can be seen that the desired improvements in the fine particle fraction of dry powder formulations containing glycopyrrolate for a period suitable for an inhalation product (e.g. 1, 2, 3 years) can be achieved by suitable conditioning, and/or by protection of the formulation from moisture, and/or by the suitable incorporation of an additive, such as a force control agent. Indeed, as the examples discussed below indicate, a combination of two or more of these measures leads to the best results. The protection of the dry powder formulation from moisture may be particularly important.

A very important advantage of the present invention is that it allows the administration of smaller doses than previously used. The reduction of the dose is made possible by the more consistent and predictable administration of the glycopyrrolate, for example, through a consistently improved fine particle fraction and fine particle dose compared to that observed in connection with the conventional formulations. Consequently, while the dose dispensed is smaller, the amount of active agent being administered is the same, with the same therapeutic effect being achieved.

The formulations of the present invention may include glycopyrrolate as the only pharmaceutically active agent. Alternatively, the formulations may include one or more further active agents, in addition to the glycopyrrolate. The additional active agents may include, for example:
1) steroid drugs such as, for example, alcometasone, beclomethasone, beclomethasone dipropionate, betamethasone, budesonide, clobetasol, deflazacort, diflucortolone, desoxymethasone, dexamethasone, fludrocortisone, flunisolide, fluocinolone, fluometholone, fluticasone, fluticasone proprionate, hydrocortisone, triamcinolone, nandrolone decanoate, neomycin sulphate, rimexolone, methylprednisolone and prednisolone;
2) antibiotic and antibacterial agents such as, for example, metronidazole, sulphadiazine, triclosan, neomycin, amoxicillin, amphotericin, clindamycin, aclarubicin, dactinomycin, nystatin, mupirocin and chlorhexidine;
3) systemically active drugs such as, for example, isosorbide dinitrate, isosorbide mononitrate, apomorphine and nicotine;
4) antihistamines such as, for example, azelastine, chlorpheniramine, astemizole, cetirizine, cinnarizine, desloratadine, loratadine, hydroxyzine, diphenhydramine, fexofenadine, ketotifen, promethazine, trimeprazine and terfenadine;
5) anti-inflammatory agents such as, for example, piroxicam, nedocromil, benzydamine, diclofenac sodium, ketoprofen, ibuprofen, heparinoid, nedocromil, cromoglycate, fasafungine and iodoxamide;
6) anticholinergic agents such as, for example, atropine, benzatropine, biperiden, cyclopentolate, oxybutinin, orphenadine hydrochloride, procyclidine, propantheline, propiverine, tiotropium, tropicamide, trospium, ipratropium bromide and oxitroprium bromide;
7) anti-emetics such as, for example, bestahistine, dolasetron, nabilone, prochlorperazine, ondansetron, trifluoperazine, tropisetron, domperidone, hyoscine, cinnarizine, metoclopramide, cyclizine, dimenhydrinate and promethazine;
8) hormonal drugs such as, for example, protirelin, thyroxine, salcotonin, somatropin, tetracosactide, vasopressin or desmopressin;
9) bronchodilators, such as salbutamol, fenoterol, formoterol and salmeterol;
10) sympathomimetic drugs, such as adrenaline, noradrenaline, dexamfetamine, dipirefin, dobutamine, dopexamine, phenylephrine, isoprenaline, dopamine, pseudoephedrine, tramazoline and xylometazoline;
11) anti-fungal drugs such as, for example, amphotericin, caspofungin, clotrimazole, econazole nitrate, fluconazole, ketoconazole, nystatin, itraconazole, terbinafine, voriconazole and miconazole;
12) local anaesthetics such as, for example, amethocaine, bupivacaine, hydrocortisone, methylprednisolone, prilocaine, proxymetacaine, ropivacaine, tyrothricin, benzocaine and lignocaine;
13) opiates, preferably for pain management, such as, for example, buprenorphine, dextromoramide, diamorphine, codeine phosphate, dextropropoxyphene, dihydrocodeine, papaveretum, pholcodeine, loperamide, fentanyl, methadone, morphine, oxycodone, phenazocine, pethidine and combinations thereof with an anti-emetic;
14) analgesics and drugs for treating migraine such as clonidine, codine, coproxamol, dextropropoxypene, ergotamine, sumatriptan, tramadol and non-steroidal anti-inflammatory drugs;
15) narcotic agonists and opiate antidotes such as naloxone, and pentazocine;
16) phosphodiesterase type 5 inhibitors, such as sildenafil; and
17) pharmaceutically acceptable salts of any of the foregoing.

Preferably, the additional active agents are pharmaceutically active agents which are known to be useful in the treatment of respiratory disorders, such as β₂-agonists, steroids, anticholinergics, phosphodiesterase 4 inhibitors, and the like. In one embodiment, the formulation of the present invention does not include formoterol.

The following examples serve to support the invention discussed above.

### Example 1

### Formulation A

The blend comprised micronised glycopyrronium bromide, with Pharmatose 150M (DMV), blend to give a 60µg dose.

### Formulation B

The blend comprised micronised glycopyrronium bromide, with Pharmatose 150M (DMV), blend to give a 120µg dose.

### Formulation C

The blend comprised micronised glycopyrronium bromide, with Pharmatose 150M (DMV), blend to give a 60µg dose.

### Formulation D

The blend comprised micronised glycopyrronium bromide, with Pharmatose 150M (DMV), blend to give a 120µg dose.

### Formulation E

The blend comprised micronised glycopyrronium bromide, with Pharmatose 150M (DMV), blend to give a 60µg dose.

### Formulation F

The blend comprised micronised glycopyrronium bromide, with Pharmatose 150M (DMV), blend to give a 120µg dose.

These powders were then loaded as the appropriate doses of 60µg and 120µg into gelatin capsules. These were then packaged and stored under selected conditions of 40°C/70% RH, 30°C/60% RH and 25°C/60% RH.

The fine particle fraction was assessed by firing the capsules from a Miat MonoHaler device into a multi stage liquid impinger, using the method defined in the European Pharmacopoeia 4th Edition 2002. Delivered dose (DD), fine particle dose (FPD) and fine particle fraction (FPF) were measured. The fine particle fraction was defined here as the mass fraction smaller than 5µm relative to the delivered dose in each case. Delivered dose (DD) was also assessed by collection into a DUSA tube using the method defined in the European Pharmacopoeia 2002. Tests were conducted at selected time-points of up to 9 months and the results are summarised in the following Tables:

### Stability of Formulation A (60µg), stored at 25°C/60% RH

| **Time (months)** | **DUSA** | **MSLI** | | |
|---|---|---|---|---|
| | **DD (µg)** | **DD (µg)** | **FPD (µg)** | **FPF (%)** |
| 0 | 52 | 53 | 24 | 45 |
| 1 | 51 | 50 | 19 | 39 |
| 2 | 55 | 51 | 20 | 39 |
| 3 | 53 | 53 | 21 | 40 |
| 6 | 46 | 50 | 20 | 40 |

### Stability of Formulation A (60µg), stored at 40°C/70% RH

| **Time (months)** | **DUSA** | **MSLI** | | |
|---|---|---|---|---|
| | **DD (µg)** | **DD (µg)** | **FPD (µg)** | **FPF (%)** |
| 0 | 52 | 53 | 24 | 45 |
| 1 | 47 | 49 | 17 | 35 |
| 2 | 46 | 46 | 14 | 31 |
| 3 | 45 | 44 | 13 | 30 |

### Stability of Formulation B (120µg), stored at 25°C/60% RH

| **Time (months)** | **DUSA** | **MSLI** | | |
|---|---|---|---|---|
| | **DD (µg)** | **DD (µg)** | **FPD (µg)** | **FPF (%)** |
| 0 | 107 | 107 | 48 | 45 |
| 1 | 102 | 104 | 45 | 43 |
| 2 | 104 | 105 | 44 | 42 |
| 3 | 110 | 111 | 44 | 40 |
| 6 | 102 | 108 | 45 | 42 |

### Stability of Formulation B (120µg), stored at 40°C/70% RH

| **Time (months)** | **DUSA** | **MSLI** | | |
|---|---|---|---|---|
| | **DD (µg)** | **DD (µg)** | **FPD (µg)** | **FPF (%)** |
| 0 | 107 | 107 | 48 | 45 |
| 1 | 105 | 104 | 37 | 36 |
| 2 | 101 | 101 | 36 | 36 |
| 3 | 97 | 97 | 27 | 28 |

### Stability of Formulation C (60µg), stored at 25°C/60% RH

| **Time (months)** | **DUSA** | **MSLI** | | |
|---|---|---|---|---|
| | **DD (µg)** | **DD (µg)** | **FPD (µg)** | **FPF (%)** |
| 0 | 50 | 49 | 17 | 34 |
| 4 | - | 49 | 16 | 32 |
| 9 | 44 | 43 | 13 | 29 |

### Stability of Formulation C (60µg), stored at 30°C/60% RH

| **Time (months)** | **DUSA** | **MSLI** | | |
|---|---|---|---|---|
| | **DD (µg)** | **DD (µg)** | **FPD (µg)** | **FPF (%)** |
| 0 | 50 | 49 | 17 | 34 |
| 9 | 43 | 45 | 12 | 27 |

### Stability of Formulation D (120µg), stored at 25°C/60% RH

| **Time (months)** | **DUSA** | **MSLI** | | |
|---|---|---|---|---|
| | **DD (µg)** | **DD (µg)** | **FPD (µg)** | **FPF (%)** |
| 0 | 97 | 105 | 32 | 31 |
| 4 | - | 99 | 28 | 29 |
| 9 | 99 | 97 | 23 | 24 |

### Stability of Formulation D (120µg), stored at 30°C/60% RH

| **Time (months)** | **DUSA** | **MSLI** | | |
|---|---|---|---|---|
| | **DD (µg)** | **DD (µg)** | **FPD (µg)** | **FPF (%)** |
| 0 | 97 | 105 | 32 | 31 |
| 9 | 99 | 98 | 24 | 25 |

### Stability of Formulation E (60µg), stored at 25°C/60% RH

| **Time (months)** | **DUSA** | **MSLI** | | |
|---|---|---|---|---|
| | **DD (µg)** | **DD (µg)** | **FPD (µg)** | **FPF (%)** |
| Release | 45 | 51 | 16 | 31 |
| Set down* | 48 | 52 | 14 | 26 |
| Set down + 4 | 45 | 47 | 10 | 20 |

| | | | | |
|---|---|---|---|---|
| * Set down was 3 months after release date | | | | |

### Stability of Formulation E (60µg), stored at 30°C/60% RH

| **Time (months)** | **DUSA** | **MSLI** | | |
|---|---|---|---|---|
| | **DD (µg)** | **DD (µg)** | **FPD (µg)** | **FPF (%)** |
| Release | 45 | 51 | 16 | 31 |
| Set down* | 48 | 52 | 14 | 26 |
| Set down + 4 | 48 | 48 | 10 | 21 |

| | | | | |
|---|---|---|---|---|
| * Set down was 3 months after release date | | | | |

### Stability of Formulation F (120µg), stored at 25°C/60% RH

| **Time (months)** | **DUSA** | **MSLI** | | |
|---|---|---|---|---|
| | **DD (µg)** | **DD (µg)** | **FPD (µg)** | **FPF (%)** |
| Release | 97 | 107 | 33 | 31 |
| Set down* | 102 | 108 | 31 | 29 |
| Set down + 4 | 99 | 105 | 24 | 23 |

| | | | | |
|---|---|---|---|---|
| * Set down was 3 months after release date | | | | |

### Stability of Formulation F (120µg), stored at 30°C/60% RH

| **Time (months)** | **DUSA** | **MSLI** | | |
|---|---|---|---|---|
| | **DD (µg)** | **DD (µg)** | **FPD (µg)** | **FPF (%)** |
| Release | 97 | 107 | 33 | 31 |
| Set down* | 102 | 108 | 31 | 29 |
| Set down + 4 | 103 | 106 | 23 | 22 |

| | | | | |
|---|---|---|---|---|
| * Set down was 3 months after release date | | | | |

It can be seen from this stability study, that all of the formulations dropped in FPF performance during the stability period when stored at 30°C/60%RH or 40°C/75% RH. However, at 25°C/60% RH, Formulations A and B had a relatively small drop in FPF compared to the other formulations, which dropped more sharply.

Formulations A and B also had a substantially greater FPF at the release compared to the other formulations, indicating large variation between these otherwise similar blends.

### Example 2

### Formulations targeted at 480µg with magnesium stearate

### Formulation 1

This blend comprised 90% of Capsulac large carrier lactose, 7.8% Sorbolac 400, 0.25% magnesium stearate and 1.92% micronised glycopyrronium bromide. The Sorbolac 400 lactose was mixed with the magnesium stearate and the micronised glycopyrronium bromide in a Kenwood Mini Chopper high shear blender for 5 minutes. At 1 minute intervals the walls of the blender were swept down to optimise mixing.

This pre-blend was then sandwiched between 2 layers of the Capsulac large carrier lactose in a capsule shaped vessel, and then Turbula blended for 1 hour at 42 rpm, followed by 10 minutes at 62 rpm to improve content uniformity.

### Formulation 2

This blend comprised 90% of Pharmatose 325 large carrier lactose, 7.8% Sorbolac 400, 0.25% magnesium stearate and 1.92% micronised glycopyrronium bromide. The Sorbolac 400 lactose was mixed with the magnesium stearate and the micronised glycopyrronium bromide in a Kenwood Mini Chopper high shear blender for 5 minutes. At 1 minute intervals the walls of the blender were swept down to optimise mixing.

This pre-blend was then sandwiched between 2 layers of the Pharmatose 325 large carrier lactose in a capsule shaped vessel, and then Turbula blended for 1 hour at 42 rpm.

### Formulations 3 and 4 (repeated)

These repeated blends comprised 90% of Pharmatose 325 large carrier lactose, 7.8% Sorbolac 400, 0.25% magnesium stearate and 1.92% micronised glycopyrronium bromide. The Sorbolac 400 lactose was mixed with the magnesium stearate and the Pharmatose 325 large carrier lactose in a GrindoMix high shear blender for 1 minute at 2000 rpm. This was left for 1 hour to reduce electrostatic charge within the powder mass.

Micronised glycopyrronium bromide was then sandwiched between 2 layers of this pre-blend in the GrindoMix, and blended for 5 minutes at 2000 rpm.

### Formulations 5 and 6 (repeated)

These repeated blends comprised 90% of Pharmatose 150 large carrier lactose, 7.8% Sorbolac 400, 0.25% magnesium stearate and 1.92% micronised glycopyrronium bromide. The Sorbolac 400 lactose was mixed with the magnesium stearate and the Pharmatose 150 large carrier lactose in a GrindoMix high shear blender for 1 minute at 2000 rpm. This was left for 1 hour to reduce electrostatic charge within the powder mass.

Micronised glycopyrronium bromide was then sandwiched between 2 layers of this pre-blend in the GrindoMix, and blended for 5 minutes at 2000 rpm, followed by a further 4 minutes to improve blend content uniformity.

### Formulation 7

This blend comprised approximately 90% of Pharmatose 150 large carrier lactose, 7.9% Sorbolac 400, 0.15% magnesium stearate and 1.9% micronised glycopyrronium bromide. The Sorbolac 400 lactose was mixed with the magnesium stearate and the Pharmatose 150 large carrier lactose in a GrindoMix high shear blender for 1 minute at 2000 rpm. This was left for 1 hour to reduce electrostatic charge within the powder mass.

Micronised glycopyrronium bromide was then sandwiched between 2 layers of this pre-blend in the GrindoMix, and blended for 9 minutes at 2000 rpm.

### Formulations targeted at 480µg without magnesium stearate

### Formulation 8

This blend comprised 90.25% of Pharmatose 325 large carrier lactose, 7.8% Sorbolac 400, and 1.92% micronised glycopyrronium bromide. The Sorbolac 400 lactose was mixed with the Pharmatose 325 large carrier lactose in a GrindoMix high shear blender for 1 minute at 2000 rpm. This was left for 1 hour to reduce electrostatic charge within the powder mass.

Micronised glycopyrronium bromide was then sandwiched between 2 layers of this pre-blend in the GrindoMix, and blended for 7 minutes at 2000 rpm.

### Formulation 9

This blend comprised 90.25% of Pharmatose 150 large carrier lactose, 7.8% Sorbolac 400, and 1.92% micronised glycopyrronium bromide. The Sorbolac 400 lactose was mixed with the Pharmatose 325 large carrier lactose in a GrindoMix high shear blender for 1 minute at 2000 rpm. This was left for 1 hour to reduce electrostatic charge within the powder mass.

Micronised glycopyrronium bromide was then sandwiched between 2 layers of this pre-blend in the GrindoMix, and blended for 7 minutes at 2000 rpm.

### Powder Testing

All formulations manufactured were assessed for satisfactory bulk powder content uniformity.

The fine particle fraction was assessed by firing the capsules from a Miat MonoHaler device into a multi stage liquid impinger (MSLI), using the method defined in the European Pharmacopoeia 4th Edition 2002. Five consecutive doses were collected under an operating air flow of 1001/min. CITDAS software was used to process the stage deposition data, and to generate delivered dose (DD), fine particle dose <5µm (FPD) and fine particle fraction <5µm (FPF).

The results are summarised in the following table.

| **Formulation** | **MSLI Performance** | | |
|---|---|---|---|
| | **DD (µg)** | **FPD (µg)** | **FPF (%)** |
| 1 | 367 | 114 | 31 |
| 2 | 385 | 86 | 22 |
| 3 | 350 | 159 | 45 |
| 4 | 384 | 179 | 46 |
| 5 | 406 | 233 | 57 |
| 6 | 420 | 229 | 54 |
| 7 | 404 | 216 | 53 |
| 8 | 390 | 148 | 38 |
| 9 | 398 | 177 | 44 |

The data show that formulations manufactured without magnesium stearate as a force control agent exhibited approximately 20% reduction in fine particle fraction and dose than the respective formulations with a force control agent. For example, Formulation 8 without a force control agent exhibited a FPF of 38%, Formulation 4 with a force control agent a FPF of 46%, Formulation 9 without a force control agent a FPF of 44% and Formulation 5 with a force control agent exhibited a FPF of 57%.

The formulations manufactured with 0.15% force control agent had a slightly lower performance than those with 0.25% force control agent (FPF of 53% compared to FPFs of 57% and 54%).

In general, the formulations in Example 2 with magnesium stearate show better FPF values than those in Example 1 without magnesium stearate.

The repeated formulations in Example 1 without magnesium stearate show greater variation in FPF than the repeated formulations in Example 2

Blend content uniformity did not seem to be affected by addition of a force control agent, but was affected by insufficient mixing, related to the lower energy blending methods or insufficient blending time. Similarly, aerosol dispersion characteristics were substantially worse for blends made with the lower energy blending process, that is, Turbula blends exhibited FPFs of 22-31% whilst high shear blends exhibited FPFs of 45-57%.

Dispersion performance for blends using Pharmatose 150M were improved over those using Pharmatose 325. This may be attributed to the increased fine lactose (i.e., % <40µm) content for the Pharmatose 150M material. Performance was consistent at 25mg and 12.5 mg capsule loadings.

Consequently, it can be concluded that the optimum performance required:
High shear blending;
Magnesium stearate content >0.05%, more preferably >0.1% but preferably not enough to create CU or toxicity problems (e.g. preferably <5%, more preferably <2%, more preferably <1%, and more preferably <0.5%); and Fine lactose content preferably >3%, more preferably >5% more preferably >8%.

### Example 3

Subsequent to this work, blends containing 400µg, 250µg and 20µg glycopyrrolate were made using the following method.

This blend comprised approximately 90% of Pharmatose 150 large carrier lactose, approximately 9% Sorbolac 400, 0.15% magnesium stearate and the micronised glycopyrronium bromide. The Sorbolac 400 lactose was mixed with the magnesium stearate and the Pharmatose 150 large carrier lactose in a GrindoMix high shear blender for 1 minute at 2000 rpm. This was left for 1 hour to reduce electrostatic charge within the powder mass.

Micronised glycopyrronium bromide was then sandwiched between 2 layers of this pre-blend in the GrindoMix, and blended for 9 minutes at 2000 rpm.

These powders were then loaded as the appropriate doses of 400µg, 250µg and 20µg into gelatin capsules, and packaged in foil pouches. These were then stored under conditions of 40°C/75% RH, 30°C/60% RH and 25°C/60% RH. The fine particle fraction was assessed by firing the capsules from a Miat MonoHaler device into a multi stage liquid impinger, using the method defined in the European Pharmacopoeia 4th Edition 2002. The fine particle fraction was defined here as the mass fraction smaller than 5µm relative to the nominal dose in each case. Selected tests were conducted at time-points of up to 52 weeks.

The data are summarised in the following tables.

| **Aerodynamic Assessment - FPF (ND) %** | |
|---|---|
| **Time (weeks)** | **400µg 40°C/75% RH Packaged in Foil Pouch** |
| 0 | 42.6 ± 1.3 |
| 4 | 30.1 ± 1.9 |
| 12 | 26.5 ± 1.4 |
| 31 | 23.9 ± 2.6 |
| **Time (weeks)** | **400µg 30°C/60% RH Packaged in Foil Pouch** |
| 0 | 42.6 ± 1.3 |
| 4 | 41.4 ± 0.9 |
| 12 | 40.7 ± 1.3 |
| 31 | 36.7 ± 1.1 |
| 42 | 38.4 ± 0.9 |
| 52 | 38.4 ± 0.8 |
| **Time (weeks)** | **400µg 25°C/60% RH Packaged in Foil Pouch** |
| 0 | 42.6 ± 1.3 |
| 12 | 42.0 ± 2.4 |
| 31 | 39.0 ± 2.5 |
| 42 | 44.9 ± 0.3 |
| 52 | 40.3 ± 1.2 |

| **Aerodynamic Assessment - FPF (ND) %** | |
|---|---|
| **Time (weeks)** | **250µg 40°C/75% RH Packaged in Foil Pouch** |
| 0 | 39.5 ± 2.0 |
| 4 | 27.6 ± 0.7 |
| 12 | 21.3 ± 1.1 |
| 31 | 19.9 ± 0.6 |
| **Time (weeks)** | **250µg 30°C/60% RH Packaged in Foil Pouch** |
| 0 | 39.5 ± 2.0 |
| 4 | 40.2 ± 1.5 |
| 12 | 35.6 ± 2.1 |
| 31 | 31.1 ± 2.5 |
| 42 | 36.9 ± 0.5 |
| 52 | 32.2 ± 4.4 |
| **Time (weeks)** | **250µg 25°C/60% RH Packaged in Foil Pouch** |
| 0 | 39.5 ± 2.0 |
| 12 | 39.2 ± 2.9 |
| 31 | 39.0 ± 1.5 |
| 42 | 39.1 ± 0.6 |
| 52 | 34.5 ± 1.1 |

| **Aerodynamic Assessment - FPF (ND) %** | |
|---|---|
| **Time (weeks)** | **20µg 40°C/75% RH Packaged in Foil Pouch** |
| 0 | 42.3 ± 1.9 |
| 4 | 20.8 ± 1.1 |
| 8 | 18.4 ± 0.9 |
| 12 | - |
| **Time (weeks)** | **20µg 30°C/60% RH Packaged in Foil Pouch** |
| 0 | 42.3 ± 1.9 |
| 4 | 35.5 ± 1.4 |
| 8 | 29.0 ± 0.3 |
| 12 | 28.8 ± 0.5 |
| **Time (weeks)** | **20µg 25°C/60% RH Packaged in Foil Pouch** |
| 0 | 42.3 ± 1.9 |
| 4 | 39.1 ± 0.4 |
| 8 | 41.2 ± 0.4 |
| 12 | 37.3 ± 0.2 |
| 23 | 36.2 ± 1.7 |
| 26 | 31.0 ± 0.5 |
| 40 | 31.8 ± 1.0 |
| 52 | 32.8 ± 1.3 |

In each case, the FPF value at the initial time-point was approximately 40%. However, in each case, the material stored at 40°C/75% RH, the FPF had dropped to below 30% after 4 weeks, and in most cases to approximately 20% after 12 weeks. The 250µg the material stored at 30°C/60% RH, the FPF had dropped to nearly 30% after 31 weeks, and the 20µg the material stored at 30°C/60% RH, the FPF had dropped to below 30% after 8 weeks.

The 250µg the material stored at 25°C/60% RH, the FPF had dropped to nearly 35% after 52 weeks, and the 20µg the material stored at 25°C/60% RH, the FPF had dropped to about 30% after 26 weeks.

Consequently, it was concluded that magnesium stearate was not providing protection from instability in these prototype formulations. A number of measures were proposed:
- To increase the magnesium stearate level
- To condition the drug by a pre-exposure to moisture
- To condition the excipients and additives by a pre-exposure to a low moisture environment
- To condition the capsules by a pre-exposure to a low moisture environment
- To employ low moisture content (e.g. HPMC) capsules
- To investigate foil aluminium overwrap.

### Example 4

In this new study, blends containing 160µg, 80µg, 40µg and 20µg glycopyrrolate are to be made using the following method. The blends comprise approximately 90% of Pharmatose 150 large carrier lactose, between approximately 9 and 9.8% Sorbolac 400, 0.15% magnesium stearate and the micronised glycopyrronium bromide. The powders are blended in a high shear mixer, in one step. These powders are preconditioned at 40% RH.

### Example 5

Blends containing 250µg and 20µg glycopyrrolate in 25mg were made using the method described in Example 3. Powders were made with 0.15% magnesium stearate. 25mg of the powders were then loaded into HPMC capsules and into gelatin capsules, and packaged in cold form aluminium foil pouches. The gelatin capsules had been pre-conditioned at 40% RH.

These were then stored under conditions of 30°C/65% RH. The fine particle fraction was assessed by firing the capsules from a Miat MonoHaler device into a multi stage liquid impinger, using the method defined in the European Pharmacopoeia 2002. Delivered dose (DD), fine particle dose (FPD) and fine particle fraction (FPF) were measured. The FPF was defined here as the mass fraction smaller than 5µm relative to the nominal dose in each case. Delivered dose (DD) was also assessed by collection into a DUSA tube using the method defined in the European Pharmacopoeia 2002.

Powders were tested at the start point and at selected timepoints of one and three months. The results of the tests are summarised below:
With 0.15% Magnesium Stearate and 250µg Glycopyrrolate

| | CT | re-micronised | re-micronised | Pre-clinical |
|---|---|---|---|---|
| **t=0** | Gelatin | HPMC | Gelatin | Gelatin |
| | 0.15% | 0.15% | 0.15% | 0.15% |
| DD | 215.9 ± 3.7 | 214.9 ± 7.7 | 203.5 ± 2.8 | 192.7 ± 6.6 |
| FPD (µg) | 106.1 ± 2.6 | 116.8 ± 6.3 | 100.5 ± 2.3 | 98.8 ± 4.9 |
| FPF (%) | 42.4 ± 1.0 | 46.7 ± 2.5 | 40.2 ± 0.9 | 39.5 ± 2.0 |
| DUSA | 204.7 ± 12.4 | N/A | N/A | 188.4 ± 16.7 |
| ***t*=1** | Gelatin | HPMC | Gelatin | Gelatin |
| **30/65** | 0.15% | 0.15% | 0.15% | 0.15% |
| | | | | **30/60** |
| DD | 196.2 ± 6.8 | 209.3 ± 2.3 | 176.1 ± 5.7 | 202.7 ± 8.4 |
| FPD (µg) | 75.2 ± 7.2 | 111.05 ± 1.6 | 66.0 ± 2.6 | 100.4 ± 3.7 |
| FPF (%) | 30.1 ± 2.9 | 44.4 ± 0.6 | 26.4 ± 1.1 | 40.2 ± 1.5 |
| DUSA | 199.4 ± 10.4 | N/A | N/A | 183.2 ± 13.6 |

With 0.15% Magnesium Stearate and 20µg Glycopyrrolate

| **t=0** | **HPMC** | **Gelatin** | **Gelatin** | **Gelatin Pre-Con** | **Gelatin** |
|---|---|---|---|---|---|
| DD | 18.1 ± 0.4 | 18.2 ± 0.3 | 17.3 ± 0.5 | 18.2 ± 0.3 | 17.0 ± 1.2 |
| FPD (µg) | 10.1 ± 0.3 | 9.6 ± 0.2 | 8.8 ± 0.3 | 8.1 ± 0.2 | 8.5 ± 0.4 |
| FPF (%) | 50.3 ± 1.3 | 47.8 ± 1.0 | 43.9 ± 1.5 | 40.5 ± 0.9 | 42.3 ± 1.9 |
| DUSA | N/A | 16.5 ± 0.6 | 16.2 ± 0.9 | N/A | 16.8 ± 0.7 |
| **t=1** | **HPMC 30/65** | **Gelatin 25/60** | **Gelatin 30/65** | **Gelatin 30/65** | **Gelatin 30/60** |
| DD | 17.6 ± 0.2 | 18.3±0.8 | 17.6 ± 0.1 | 15.2 ± 0.1 | 16.9 ± 0.5 |
| FPD (µg) | 9.4 ± 0.2 | 8.6 ± 0.5 | 7.7 ± 0.1 | 6.5 ± 0.2 | 7.1 ± 0.3 |
| FPF (%) | 46.8 ± 1.0 | 42.9± 2.5 | 38.7 ± 0.5 | 32.3 ± 0.9 | 35.5 ± 1.4 |
| DUSA | N/A | 17.4 ± 1.4 | 16.8 ± 0.7 | N/A | 16.5 ± 1.4 |
| **t=3 30/65** | **HPMC 30/65** | **Gelatin 25/60** | **Gelatin 30/65** | **Gelatin 30/65** | **Gelatin 30/60** |
| DD | 17.2 ± 0.2 | 17.8 ± 1.8 | 18.3 ± 0.1 | 16.2 ± 0.4 | 16.4 ± 0.4 |
| FPD (µg) | 9.1 ± 0.1 | 7.9 ± 0.3 | 7.5 ± 0.1 | 6.1 ± 0.2 | 5.8 ± 0.1 |
| FPF (%) | 45.8 ± 0.3 | 39.6 ± 1.3 | 37.3 ± 0.7 | 30.7 ± 0.8 | 28.8 ± 0.5 |
| DUSA | N/A | 16.0 ± 0.7 | 16.7 | N/A | 15.7 ± 0.6 |

In each case using HPMC capsules, the FPF started at a higher level relative to the equivalent powders in gelatin capsules and remained high (at least 44%) over the 3 month period. In each case using gelatin capsules, the FPF started at the slightly lower level than had been seen with HPMC capsules, but also in several instances dropped significantly over the 3 month period to 30% or below.

This study supports the benefit of using a low moisture capsule in resolving the problem presented by micronised glycopyrrolate as outlined above.

This study also supports our belief that the basic aerosolisation process is more efficient with HPMC capsules compared to gelatin capsules. We believe this is due to the improved piercing of holes formed in the HPMC capsules.

### Example 6

As an alternative device, a prototype system termed the GyroHaler (as briefly described above) was used. This device protects the formulation from moisture by containing the powder within pre-metered foil blister strips. Consequently, no moisture source is available to the powder providing integrity of the seals is maintained.

In this study, blends containing 250µg in 15mg or 20µg in 25mg glycopyrrolate were made using the following method. This blend comprised approximately 90% of Pharmatose 150 large carrier lactose, between approximately 9 and 10% Sorbolac 400, 0.15% magnesium stearate and the micronised glycopyrronium bromide. The powders were blended in a high shear mixer, in one step.

The powder was metered into each foil blister which was subsequently sealed with a foil lid. The device was actuated by allowing a piercing head to pierce the blister lid. The powders were then drawn through the mouthpiece and into a multi stage liquid impinger, at 60 l/min, using the method defined in the European Pharmacopoeia 2002. In each case, the fine particle fractions were between 45 and 50%. The fine particle fraction was defined here as the mass fraction smaller than 5µm relative to the delivered dose in each case.

### Example 7

The effect of conditioning on micronised glycopyrrolate was investigated. An initial batch of glycopyrrolate 'A' was micronised at 9.8 bar with feed rate of 0.2 kg/hour. This material was then conditioned on a tray at 25°C/60% RH, with or without agitation/turning. Each of these powders was sized by Sympatec. The powders were then formulated using the method outlined in Example 4, as 20µg dose in 25mg powder with 0.15% magnesium stearate and loaded into gelatin capsules. The fine particle fraction was assessed by firing the capsules from a Miat MonoHaler device into a multi stage liquid impinger, using the method defined in the European Pharmacopoeia 4th Edition 2002. The fine particle fraction was defined here as the mass fraction smaller than 5µm relative to the nominal dose.

A second batch of glycopyrrolate 'B' was micronised at 9.8 bar with feed rate of 0.3 kg/hour. This powder was sized by Sympatec. The powder was then formulated using the method outlined in Example 4, as 20µg dose in 25mg powder with 0.15% magnesium stearate and loaded into gelatin capsules. The fine particle fraction was assessed by firing the capsules from a Miat MonoHaler device into a multi stage liquid impinger, using the method defined in the European Pharmacopoeia 4th Edition 2002. The fine particle fraction was defined here as the mass fraction smaller than 5µm relative to the nominal dose.

A third batch of glycopyrrolate 'C' was micronised at 9.8 bar with feed rate of 0.4 kg/hour. This material was then conditioned on a tray at 25°C/60% RH, with or without agitation/turning. Each of these powders was sized by Sympatec. The powders were then formulated using the method outlined in Example 4, as 20µg dose in 25mg powder with 0.15% magnesium stearate and loaded into gelatin capsules. The fine particle fraction was assessed by firing the capsules from a Miat MonoHaler device into a multi stage liquid impinger, using the method defined in the European Pharmacopoeia 4th Edition 2002. The fine particle fraction was defined here as the mass fraction smaller than 5µm relative to the nominal dose.

A fourth batch of glycopyrrolate 'D' was micronised at 8.8 bar with feed rate of 0.4 kg/hour. This powder was sized by Sympatec. The powder was then formulated using the method outlined in Example 4, as 20µg dose in 25mg powder with 0.15% magnesium stearate and loaded into gelatin capsules. The fine particle fraction was assessed by firing the capsules from a Miat MonoHaler device into a multi stage liquid impinger, using the method defined in the European Pharmacopoeia 4th Edition 2002. The fine particle fraction was defined here as the mass fraction smaller than 5µm relative to the nominal dose.

A fifth batch of glycopyrrolate 'E' was micronised at 7.8 bar with feed rate of 0.4 kg/hour. This material was then conditioned on a tray at 25°C/60% RH, with or without agitation/turning. Each of these powders was sized by Sympatec. The powders were then formulated using the method outlined in Example 4, as 20µg dose in 25mg powder with 0.15% magnesium stearate and loaded into gelatin capsules. The fine particle fraction was assessed by firing the capsules from a Miat MonoHaler device into a multi stage liquid impinger, using the method defined in the European Pharmacopoeia 4th Edition 2002. The fine particle fraction was defined here as the mass fraction smaller than 5µm relative to the nominal dose.

The results from each of the tests on batches A to E are summarised below. Batches A1, C1 and E1 were not conditioned. Batches A2, C2 and E2 were conditioned at 25°C/60% RH and batches A3, C3 and E3 were conditioned at 25°C/60% RH with turning.

| | **GP (bar)** | **Feed rate (kg/h)** | **D₉₀ µm** | **D₅₀ µm** | **D₁₀ µm** | **T=0** | | **T=2 wks** | |
|---|---|---|---|---|---|---|---|---|---|
| | | | | | | **% FPD** | **MMAD (µm)** | **%FPD** | **MMAD (µm)** |
| A1 | 9.8 | 0.2 | 3.68 | 1.95 | 0.81 | 34.7 | 2.8 | 30.5 | 3.2 |
| A2 | | | 10.02 | 3.89 | 1.22 | ND | ND | ND | ND |
| A3 | | | 9.78 | 4.03 | 1.24 | 34.5 | 2.8 | 31.5 | 3.1 |
| B | 9.8 | 0.3 | 4.25 | 2.14 | 0.85 | ND | ND | ND | ND |
| C1 | 9.8 | 0.4 | 4.83 | 2.41 | 0.95 | 39.9 | 3.9 | 35.5 | 4.0 |
| C2 | | | 7.84 | 3.76 | 1.24 | 39.2 | 3.3 | 34.0 | 3.6 |
| C3 | | | 8.23 | 3.97 | 1.24 | 39.9 | 3.2 | 37.9 | 3.4 |
| D | 8.8 | 0.4 | 4.86 | 2.44 | 0.98 | 37.9 | 3.2 | 31.9 | 3.6 |
| E1 | 7.8 | 0.4 | 4.88 | 2.47 | 1.01 | 39.9 | 3.3 | 33.3 | 3.4 |
| E2 | | | 7.08 | 3.61 | 1.28 | ND | ND | ND | ND |
| E3 | | | 7.85 | 3.79 | 1.23 | 38.7 | 3.2 | 32.7 | 3.7 |

### Micronisation Trial Results

The Malvern particle size data show that particle size can be influenced by powder feed rate. The relationship between feed rate and particle size obtained is probably non-linear. So, depending on how close the operation is to the most sensitive conditions, an effect may or may not be seen. Here an effect is seen. Similarly, an effect would be expected with milling pressure, but in contrast this data suggest between 8 and 10 bar it appears to be above the pressure-sensitive conditions, so little change in d50 is seen at constant feed rate.

In each case, the Malvern d50s grow significantly on exposure to moisture, doubling diameter which probably represents formation of hard agglomerates of ~8 primary particle equivalents. This is consistent with formation of solid bridges, as is anticipated from the amorphous to crystalline transition. However, it is interesting to note that the MMADs produced from dispersion testing the formulations do not mirror such growth when comparing the formulations.

It is suggested that the Malvern disperser has not been strong enough to destroy these solid bridges down to primary particles. However, the milling action occurring when these drug materials were blended in the high shear mixer with large lactose particles contained in the Pharmatose 150M can be quite substantial (i.e. larger than approximately 50µm), and may well be sufficient to return the drug agglomerates to its primary size, at least transiently.

### Example 8

### Mechanofused Glycopyrrolate with Magnesium Stearate

### Blend 1: Micronised Glycopyrrolate Bromide + 5% Magnesium Stearate

A further study was conducted to look at the mechanofusion of the drug with a force control agent. The force control agent used was magnesium stearate. The blends were prepared by using the Hosokawa AMS-MINI system (Hosokawa Micron Ltd), blending 95% micronised glycopyrrolate bromide with 5% magnesium stearate for 60 minutes at approximately 4000 rpm.

This powder was kept stored in a sealed bottle for approximately 4 years. In order to determine the performance of this material after this time, blends were produced and a selected formulation tested for aerosol performance.

As the name suggests, mechanofusion is term referring to a dry coating process designed to mechanically fuse a guest material onto a host material. The process was conducted here in order to achieve a drug powder which was less susceptible to formation of solid bridges and related instability such as via re-crystallisation over time.

For mechanofusion the guest material is generally smaller and/or softer than the host. The equipment used for mechanofusion are distinct from alternative mixing and milling techniques in having a particular interaction between one or more inner elements and a vessel wall, and are based on providing energy by a controlled and substantial compressive force. Suitable equipment for mechanofusion includes the MechanoFusion range of systems made by Hosokawa, the Cyclomix range of systems made by Hosokawa, the Nobilta systems made by Hosokawa, the Hybridiser made by Nara, and all related such systems. Mills such as ball mills may also be used for this purpose, as can pin mills, disc mills, mortar mills and other such mills. Jet mills may also be used.

In one embodiment, the powder is compressed between the fixed clearance of the drum wall and one or more inner elements with high relative speed between drum and element. The inner wall and the curved element together form a gap or nip in which the particles are pressed together. As a result, the particles experience very high shear forces and very strong compressive stresses as they are trapped between the inner drum wall and the inner element. The particles are pressed against each other with enough energy to locally heat and soften, break, distort, flatten and wrap the additive particles around the core particle to form a coating. The energy is generally sufficient to break up agglomerates and some degree of size reduction of both components may occur.

An especially desirable aspect of the described processes is that the additive material becomes deformed in the milling and may be smeared over or fused to the surfaces of the active particles.

For the purposes of this method, all forms of co-milling are encompassed, including methods similar or related to those methods described above. For example, methods similar to MechanoFusion are encompassed, such as those utilizing very high speed rotors (i.e. 1000 to 50000rpm) with elements sweeping the internal surfaces of the vessels with small gaps between wall and element (i.e. 0.1mm to 20mm).

### Blend 2: Mechanofused Fine Lactose +1% Magnesium Stearate

Batches were prepared by combining 198g Sorbolac 400 (Meggle) lactose with 2g magnesium stearate. The Cyclomix (Hosokawa Micron Ltd, set with a 1mm gap) was set running at 200rpm. Half the lactose was added followed by the magnesium stearate and the remaining lactose. The speed was slowly increased to run at 2000rpm for 10 minutes.

### Blend 3: Mechanofused Large Carrier lactose + 0.12% Magnesium Stearate

Batches were prepared by combining 199.76g Respitose SV003 (DMV) lactose plus 0.24g magnesium stearate. The Cyclomix (Hosokawa Micron Ltd, set with a 1mm gap) was set running at 200rpm. Half the lactose was added followed by the magnesium stearate and the remaining lactose. The speed was slowly increased to run at 2000rpm for 10 minutes.
A combination of Blends 1, 2 and 3 comprising treated drug, fine and coarse carrier lactose was prepared as follows: 90% Blend 3 + 9.5% Blend 2 + 0.5% Blend 1. The powders were layered in a glass vessel. The vessel was sealed and the powders blended in a Turbula tumbling blender at 37rpm for 10 minutes.

10 capsules were filled with 25mg ± 5mg of this powder in order to target a dose of approximately 120µg of glycopyrrolate. All 10 capsules were then were fired from a MonoHaler (Miat) at 70 l/min into a TSI. Stages 1 and 2 were analysed by UV spectroscopy at 220nm. An average fine particle fraction of 40% was calculated for this blend, where the fraction was calculated as that less than 5µm.

This demonstrated that the drug powder has exhibited excellent stability over 4 year's storage, and was able to produce a good fine particle cloud on aerosolisation from an inhaler.

### Conditioning of micronised drug particles

The above example illustrates how micronised drug particles may be conditioned, in order to reduce the surface non-crystalline material present. The conditioning involves exposing the glycopyrrolate to humid conditions of 30-100 RH, preferably 40-95 RH, 45-95 RH or 50-90 RH. The glycopyrrolate powder is preferably placed on a tray for this step and the powder is preferably agitated or turned to ensure that all of the particles are equally exposed to the humid atmosphere. The turning or agitating also helps to avoid or reduce agglomeration of the particles during the conditioning process. The conditioning preferably takes place over a period of at least about 10 minutes, at least about 20 minutes, at least about 30 minutes, at least about 40 minutes, at least about 50 minutes, at least about 1 hour, at least about 2, 3, 4, 5, 6, 8, 10, 12, 14, 18, 24, 36 or 48 hours.

Conditioning may also be achieved in a variety of alternative ways. Some further general approaches are outlined below.

Particles extracted from the dynamic micronisation process are collected and may be transported to a suitable vessel for conditioning at a controlled humidity. In such a system, preferably the particles are all exposed to the humidity for sufficient time for the water absorption and for the re-crystallisation process to occur. Preferably all the powder remains in the vessel from start to finish of this process.

If the micronisation process itself were conducted using gas at elevated humidity, this exposure would be less easy to control. While powder could be conditioned in the collection system, powder added at the end of the process would have less time to condition than powder added at the start.

The Relative Humidity may be in the range 30 to 100%, more preferably 40 to 95%, more preferably 45 to 95% and most preferably 50 to 90%. The temperature may be varied, and preferably be in the range 5°C to 90°C, more preferably 10°C to 50°C.

The vessel may be for example a tray, or a bag. It should allow suitable exposure of the powder surface to the moisture applied from the atmosphere. The powder may be agitated or not agitated. If the powder is placed on a tray, it is preferably spread evenly in a relatively thin layer over the tray.

As an alternative, the micronised powder may be transferred to a system which creates a fluidised bed of the micronised powder. Such systems are known in the art. The micronised powder is difficult to fluidise alone, and consequently fluidisation media are advantageously added, such as metal, plastic, glass or ceramic beads, typically with diameters in the range 100µm to 5mm.

A fluidised bed aerosol technique for this purpose could be one as described by Morton et al (J. Aerosol Science, Vol. 26, No.3, p353 and references therein).

The following numbered embodiments further describe the current invention.
a) A dry powder formulation comprising glycopyrrolate, wherein the formulation is stable over a period of at least 1 year under normal conditions.
b) A dry powder formulation as disclosed in embodiment (a), wherein the formulation is stable over a period of at least 2 years.
c) A dry powder formulation as disclosed in embodiment (a), wherein the formulation is stable over a period of at least 3 years.
d) A dry powder formulation as disclosed in any one of the preceding embodiments, wherein instability of the formulation is indicated by the formation of hard agglomerates.
e) A dry powder formulation as disclosed in any one of the preceding embodiments, wherein stability of the formulation is indicated by consistent good fine particle fraction or fine particle dose values.
f) A dry powder formulation as disclosed in embodiment (e), wherein the fine particle fraction of the powder is consistently at least about 30%.
g) A dry powder formulation as disclosed in embodiment (f), wherein the fine particle fraction of the powder is consistently at least about 40%.
h) A dry powder formulation as disclosed in any one of the preceding embodiments, wherein stability of the formulation is achieved by preventing or reducing the uptake of moisture by the formulation.
i) A dry powder formulation as disclosed in any one of the preceding embodiments, wherein the glycopyrrolate is micronised.
j) A dry powder formulation as disclosed in embodiment (i), wherein the glycopyrrolate is conditioned during or following micronisation to reduce the tendency of the formulation to absorb moisture.
k) A dry powder formulation as disclosed in embodiment (j), wherein the conditioning involves controlled exposure of the glycopyrrolate to moisture.
l) A dry powder formulation as disclosed in embodiments (j) or (k), wherein the conditioning involves disruption of any solid bridges formed during or following micronisation.
m) A dry powder formulation as disclosed in any one of the preceding embodiments, wherein the formulation further comprises a force control agent which is capable of reducing cohesion between the fine particles in the formulation.
n) A dry powder formulation as disclosed in embodiment (m), wherein the force control agent also acts as a surfactant.
o) A dry powder formulation as disclosed in embodiments (m) or (n), wherein the force control agent prevents the ingress of moisture into the formulation.
p) A dry powder formulation as disclosed in embodiment (m), wherein the force control agent is magnesium stearate, one or more amino acids or their derivatives, lecithin or phospholipids
q) A dry powder formulation as disclosed in embodiment (p), wherein the amino acid is leucine, lysine, arginine, histidine or cysteine, or derivatives thereof.
r) A dry powder formulation as disclosed in any one of the preceding embodiments, wherein the formulation is stored in packaging made from a material which has a moisture content of less than 10%.
s) A dry powder formulation as disclosed in embodiment (r), wherein the packaging material has a moisture content of less than 5%.
t) A dry powder formulation as disclosed in embodiment (r), wherein the packaging material has a moisture content of less than 3%.
u) A dry powder formulation as disclosed in embodiments (r), (s) or (t), wherein the packaging is an HPMC capsule.
v) A dry powder formulation as disclosed in any one of the preceding embodiments, wherein the formulation is stored in packaging which is capable of preventing the ingress of moisture form external sources.
w) A dry powder formulation as disclosed in embodiment (v), wherein the packaging is a foil sealed blister.
x) A dry powder formulation as disclosed in any one of embodiments (r) to (w), wherein the packaging is itself protected from the ingress of moisture from external sources.
y) A dry powder inhaler device comprising a dry powder formulation as disclosed in any one of the preceding embodiments.
z) A method of preparing a dry powder formulation as disclosed in any one of embodiments (a) to (y), wherein the glycopyrrolate is micronised and the micronisation process is performed under conditions which reduce the formation of amorphous material and/ or wherein the glycopyrrolate is conditioned to reduce the amorphous material content.
aa) A method as disclosed in embodiment (z), wherein the conditioning involves controlled exposure of the glycopyrrolate to moisture.
bb) A method as disclosed in embodiments (z) and (aa), wherein the conditioning involves disruption of any solid bridges formed during or immediately following micronisation.
cc) A method as disclosed in any one of embodiments (z) to (bb), wherein a force control agent which is capable of reducing cohesion between the fine particles in the formulation is added to the glycopyrrolate.

## Claims

1. A dry powder formulation suitable for inhalation comprising glycopyrrolate and magnesium stearate, wherein the glycopyrrolate is micronised and then undergoes a conditioning step, which step includes exposure to humid conditions of 30-100% RH at temperatures between 5°C to 90°C for at least 48 hours.

2. The formulation of Claim 1, wherein the conditioning step involves exposing the glycopyrrolate to humid conditions of 50-90% RH; and/or
wherein the conditioning involves exposing the glycopyrrolate to temperatures between 10 to 50°C.

3. The formulation according to Claim 1 or Claim 2 wherein the magnesium stearate content in the formulation is >0.05% and <5%.

4. The formulation according to any one of the preceding claims, wherein the formulation further comprises one or more further active agents in addition to glycopyrrolate.

5. The formulation according to Claim 4, wherein the one or more further active agents are selected from a β-2 agonist, steroids, anticholinergics and phosphodiesterase inhibitors.

6. The formulation according to Claim 1, Claim 4 or Claim 5, wherein the formulation does not include formoterol.

7. The formulation according to Claims 1 to 3, provided that the formulation does not contain an active agent selected from a β-2 agonist, steroids, anticholinergics and phosphodiesterase inhibitors.

8. The formulation according to Claim 7, wherein the formulation does not contain one or more further active agents in addition to glycopyrrolate.

9. The formulation as claimed in any one of the preceding claims, wherein the formulation is stored in packaging made from a material which has a moisture content of less than 10%.

10. The formulation of Claim 9, wherein the formulation is stored in a hydroxypropyl methylcellulose capsule.

11. A formulation as claimed in any one of the preceding claims wherein the magnesium stearate is smeared over or fused to the surface of the micronised glycopyrrolate.

12. A formulation as claimed in any one of the preceding claims wherein the magnesium stearate is co-milled with the glycopyrrolate, for example, wherein the magnesium stearate is jet-milled with the glycopyrrolate.

13. A formulation as claimed in any one of Claims 10 to 12, wherein the conditioning step occurs after the addition of magnesium stearate to the micronised glycopyrrolate.

14. A formulation as claimed in any one of the preceding claims, wherein, for the conditioning step, the micronised glycopyrrolate is placed:
a) on a tray and optionally subjected to agitation; or
b) in a fluidised bed system, optionally in the presence of fluidisation media.

15. A dry powder formulation as claimed in any one of the preceding claims, wherein the formulation is stored in packaging which is capable of preventing the ingress of moisture from external sources, such as wherein the packaging is itself protected from ingress of moisture from external sources.

16. A dry powder inhaler device comprising a dry powder formulation as claimed in any one of the preceding claims.
